# FASCICULE DE BREVET EUROPEEN

(11) **EP 3 849 627 B1**
(45) Date de publication et mention de la délivrance du brevet: **07.06.2023**
(21) Numéro de dépôt: 19787019.9
(22) Date de dépôt: 10.09.2019
(51) Int. Cl.: A61M 1/00, A61M 1/36

(54) **DISPOSITIF DE FILTRATION D'UN FLUIDE SANGUIN COMPORTANT DES RÉSIDUS OSSEUX**
VORRICHTUNG ZUM FILTERN EINER KNOCHENRÜCKSTÄNDE ENTHALTENDEN BLUTFLÜSSIGKEIT
DEVICE FOR FILTERING A BLOOD FLUID COMPRISING BONE RESIDUES

(30) Priorité: 11.09.2018 FR 1858141
(43) Date de publication de la demande: 21.07.2021
(73) Titulaire: Spinedust, 13008 Marseille (FR)
(72) Inventeur: GRAZIANI, Noël, 13004 MARSEILLE (FR)
(74) Mandataire: Germain Maureau
(86) Numéro de dépôt international: PCT/FR2019/052084
(87) Numéro de publication internationale: WO 2020/053517

(56) Documents cités:
- DE-A1-102012 012 597
- US-A1- 2005 189 288
- US-A1- 2007 225 665
- US-A1- 2015 090 652

## Description

La présente invention concerne un dispositif de traitement des résidus osseux prélevés sur un patient et destinés à une greffe autogène. Plus précisément, l'invention concerne un dispositif de filtration par aspiration permettant de séparer les résidus osseux présent dans un fluide sanguin lors d'une intervention de chirurgie notamment de décompression et d'arthrodèse du rachis. L'étendue de l'invention est limitée par la revendication 1.

La greffe autogène de résidus osseux est une technique qui consiste à prélever des petites quantités d'os sur un patient pour les greffer sur le même patient afin de combler ou de remodeler un os fraisé par nécessité durant l'opération.

Cependant, dans la collecte d'os autogène classique une grande partie des résidus osseux résultant du fraisage d'un os est perdue par l'aspiration du fluide sanguin aspiré dans la zone d'intervention du chirurgien.

Une compresse peut être utilisée pour la collecte de ces résidus osseux. Un autre moyen de collecte consiste en l'utilisation d'une curette pour prélever manuellement ces résidus. Les résidus osseux sont préférentiellement nettoyés avant la greffe autologue. Dans tous les cas, les moyens de collecte de ces résidus nécessitent de mobiliser un ou plusieurs opérateurs.

L'état de la technique peut également être illustré par les enseignements du document US 2007/225665 qui divulgue un dispositif de filtration pour une filtration d'un fluide sanguin comportant des résidus osseux, comprenant un récipient de fluide sanguin fermé par un couvercle et relié à un dispositif d'aspiration, et un moyen de filtration logé à l'intérieur du récipient. Dans ce document US 2007/225665, le moyen de filtration est un disque filtrant qui délimite deux chambres, une conduite d'entrée est fixée sur le disque filtrant pour l'entrée du fluide sanguin comportant des résidus osseux à l'intérieur de l'une des chambres, et une conduite de sortie est reliée au dispositif d'aspiration et débouche dans l'autre chambre. Ce dispositif de filtration est cependant peu efficace car la filtration est lente et dépend fortement de la pression d'aspiration.

La présente invention vise à pallier à au moins l'un de ces inconvénients en proposant un dispositif de filtration peu encombrant permettant un traitement des résidus osseux aisé et rapide, tout en préservant l'intégrité de ces résidus sans sortir du champ opératoire.

A cet effet, l'invention a pour objet un dispositif de filtration pour une filtration d'un fluide sanguin comportant des résidus osseux, ledit dispositif de filtration comprenant :
- un récipient et un couvercle agencé pour une fermeture du récipient, ce récipient et ce couvercle délimitant entre eux une chambre d'aspiration du fluide sanguin dans laquelle débouchent au moins une ouverture de sortie apte à être reliée à un dispositif d'aspiration du fluide sanguin, et une ouverture d'entrée pour une entrée du fluide sanguin comportant des résidus osseux ;
- un moyen de filtration logé dans le récipient pour la collecte des résidus osseux,
ledit dispositif de filtration étant remarquable en ce que le moyen de filtration est pourvu d'un caisson filtrant qui comprend une paroi de fond et un tamis périphérique à travers lequel le fluide sanguin est filtré et par lequel les résidus osseux sont retenus ;
et en ce qu'il comprend un moyen de centrifugation conformé pour générer un effet centrifuge du fluide sanguin à l'intérieur du caisson filtrant

Ainsi, l'invention se propose de créer un effet centrifuge du fluide sanguin à l'intérieur d'un caisson filtrant, procurant l'avantage de projeter le fluide sanguin à travers le tamis périphérique, et donc de favoriser et accélérer une filtration du fluide sanguin comportant les résidus osseux, avec les résidus osseux qui sont retenus par le tamis périphérique, et éventuellement aussi par la paroi de fond du caisson filtrant par sédimentation.

Dans une réalisation avantageuse, le moyen de centrifugation comprend une hélice et le dispositif d'aspiration du fluide sanguin qui entraine en rotation ladite hélice.

Autrement dit, l'effet centrifuge du fluide sanguin est procuré par une hélice qui est mise en mouvement par le dispositif d'aspiration du fluide sanguin, ce qui permet avantageusement d'exploiter comme source d'énergie - pour l'effet centrifuge - le dispositif d'aspiration du fluide sanguin qui est classiquement présent dans une salle d'opération.

Dans un premier mode de réalisation de l'invention, le moyen de centrifugation comprend un moyen d'entraînement couplé au moyen de filtration pour un entraînement en rotation du moyen de filtration.

Ainsi, dans ce premier mode de réalisation, l'effet centrifuge est procuré par la mise en rotation du moyen de filtration lui-même, et donc du caisson filtrant.

Selon une caractéristique de ce premier mode de réalisation, et dans le cas où le moyen de centrifugation comprend une hélice, alors l'hélice est couplée en rotation au caisson filtrant de sorte que le moyen d'entraînement entraîne en rotation le caisson filtrant.

Ainsi, le dispositif d'aspiration du fluide sanguin fait tourner l'hélice et cette hélice fait elle-même tourner le caisson filtrant pour procurer l'effet centrifuge recherché.

Selon une possibilité, le moyen d'entraînement comprend un arbre d'accouplement traversant une paroi de fond du récipient pour être couplé en rotation, d'une part, au caisson filtrant et, d'autre part, à l'hélice. Ainsi, cette hélice est externe au récipient.

Selon une autre possibilité, l'hélice est comprise dans un support pour un guidage en rotation de l'hélice, le support étant être solidarisé au récipient.

Avantageusement, le support est solidarisé au récipient par tout moyen de fixation, préférentiellement par ultrasons.

Selon une caractéristique avantageuse, le support délimite une chambre d'entraînement de l'hélice, la chambre d'aspiration du fluide sanguin et la chambre d'entraînement étant hermétique l'une par rapport à l'autre.

Selon une autre caractéristique avantageuse, le support comporte latéralement un orifice d'entrée d'air et un orifice de sortie d'air en regard l'un de l'autre pour permettre l'entraînement en rotation de l'hélice, l'orifice de sortie d'air étant relié au dispositif d'aspiration du fluide sanguin, de sorte que ce dispositif d'aspiration du fluide sanguin génère un flux dans le support qui va faire tourner l'hélice.

Selon une particularité de ce premier mode de réalisation de l'invention, le récipient comprend une ouverture de sortie du fluide sanguin, l'ouverture de sortie et l'orifice de sortie d'air du support reliés à une même valve du dispositif d'aspiration du fluide sanguin.

Selon une caractéristique de ce premier mode de réalisation de l'invention, l'ouverture d'entrée est ménagée sur le couvercle, pour facilement diriger le fluide sanguin par le dessus à l'intérieur du caisson filtrant.

Dans un second mode de réalisation de l'invention, et dans le cas où le moyen de centrifugation comprend une hélice, l'hélice est disposée à l'intérieur du caisson filtrant en étant entourée par le tamis périphérique et l'hélice est montée rotative selon un axe de rotation.

Ainsi, dans ce second mode de réalisation, l'effet centrifuge est procuré par la mise en rotation de l'hélice (et pas du caisson filtrant comme dans le cas du premier mode de réalisation).

Selon une caractéristique de ce second mode de réalisation, le caisson filtrant est statique à l'intérieur du récipient et l'ouverture d'entrée est ménagée sur le récipient en regard d'une fenêtre ménagée dans le tamis périphérique, pour faire entrer le fluide sanguin à l'intérieur du caisson filtrant par le côté (ou radialement), en direction de l'hélice disposée dans ce caisson filtrant, contribuant ainsi à faire tourner cette hélice.

Avantageusement, l'ouverture d'entrée s'étend selon un axe d'entrée intersectant l'axe de rotation de l'hélice et formant avec cet axe de rotation un premier plan médian, ce premier plan médian étant orthogonal à un second plan médian incluant l'axe de rotation, et la ou les ouvertures de sortie sont ménagées sur le récipient en étant déportées par rapport au premier plan médian, avec l'ouverture d'entrée et la ou les ouvertures de sortie disposées de part et d'autre du second plan médian.

Cette conformation est avantageuse pour créer un courant, entre l'ouverture d'entrée et la ou les ouvertures de sortie, qui va passer latéralement (ou tangentiellement) par l'hélice afin de la faire tourner.

Selon une variante, une ouverture de sortie s'étend selon un axe de sortie intersectant l'axe de rotation de l'hélice et formant avec cet axe de rotation un premier plan médian, ce premier plan médian étant orthogonal à un second plan médian incluant l'axe de rotation, et l'ouverture d'entrée est ménagée sur le récipient en étant déportée par rapport au premier plan médian, avec l'ouverture d'entrée et la ou les ouvertures de sortie disposées de part et d'autre du second plan médian.

Selon une particularité de ce second mode de réalisation de l'invention, sont prévues deux ouvertures de sortie, dont une ouverture de sortie supérieure disposée au-dessus de la paroi de fond du caisson filtrant, et une ouverture de sortie inférieure disposée en-dessous de la paroi de fond du caisson filtrant.

Selon une possibilité, l'ouverture de sortie supérieure et l'ouverture de sortie inférieure sont chacune reliée à une même valve du dispositif d'aspiration du fluide sanguin.

Selon une autre possibilité, la paroi de fond du caisson filtrant présente une forme générale de cuvette en creux, en particulier une cuvette de forme générale tronconique ou hémisphérique, favorsiant la sédimentation des débris osseux.

Selon une autre possibilité, l'hélice comprend un arbre central centré sur l'axe de rotation et depuis lequel s'étendent radialement des pales, où ledit arbre central présente une extrémité inférieure montée pivotante sur la paroi de fond du caisson filtrant.

Dans une réalisation particulière, l'arbre central de l'hélice présente une extrémité supérieure montée pivotante sur un plateau supérieur fixé sur le tamis périphérique du caisson filtrant.

Selon une variante, cette extrémité supérieure de l'arbre central est montée pivotante sur le couvercle.

Selon une particularité, le tamis est formé de mailles ayant une dimension comprise entre 100 micromètres et 1000 micromètres.

Plus préférentiellement, les mailles du tamis ont une dimension comprise entre 500 micromètres et 900 micromètres.

Dans une réalisation particulière, la paroi de fond du caisson filtrant est pourvue d'une ou plusieurs zones de tamisage.

Selon une particularité, la ou les zones de tamisage de la paroi de fond du caisson filtrant sont formées de mailles ayant une dimension comprise entre 100 micromètres et 1000 micromètres, et de préférence entre 500 micromètres et 900 micromètres.

Bien évidemment, l'invention ne se limite pas aux seules formes de réalisation de l'architecture décrites dans ces différents modes de réalisation à titre d'exemples sans toutefois sortir du contexte de l'invention.

D'autres aspects, buts et avantages de l'invention apparaîtront à la lecture de la description détaillée suivante de formes de réalisation préférées de celle-ci, donnée à titre d'exemple non limitatif et faite en référence au dessin annexé sur lequel :
- la figure 1 représente une vue schématique en perspective et en éclatée d'un dispositif de filtration selon un premier mode de réalisation de l'invention ;
- la figure 2 représente une vue schématique en perspective et en transparence d'un dispositif de filtration selon un second mode de réalisation de l'invention ;
- la figure 3 représente une vue schématique de dessus du dispositif de filtration de la figure 2 sans son couvercle ; et
- la figure 4 représente une vue schématique en perspective et en éclatée du dispositif de filtration de la figure 2.

La description détaillée qui suit porte sur deux dispositifs de filtration 1, 100 conformes à l'invention, où ces dispositifs de filtration 1, 100 sont présentés et décrits à titre d'exemples illustratifs et non limitatifs de l'invention. Dans la description qui suit, et sur l'ensemble des figures, des références identiques ou analogues désignent des organes, ensemble d'organes, moyens fonctionnels ou moyens structurels identiques ou analogues pour les différents dispositifs de filtration 1, 100.

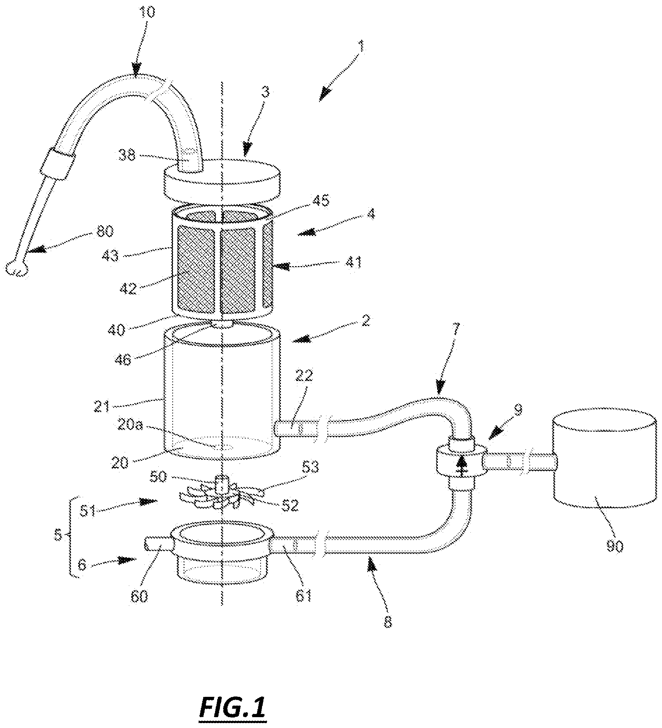

La suite de la description porte sur le premier mode de réalisation de la Figure 1, sur laquelle est représenté un dispositif de filtration 1 (appelé par la suite premier dispositif de filtration 1) pour une filtration d'un fluide sanguin comportant des résidus osseux selon un premier mode de réalisation de l'invention.

Le premier dispositif de filtration 1 comprend :
- un récipient 2 pour réceptionner le fluide sanguin,
- un couvercle 3 agencé pour une fermeture du récipient 2,
- un moyen de filtration 4 logé dans le récipient 2 pour la collecte des résidus osseux et
- un moyen de centrifugation conformé pour générer un effet centrifuge du fluide sanguin à l'intérieur du récipient 2.

Le récipient 2 est formé d'une paroi de fond 20 et d'une paroi latérale 21 (ou paroi périphérique) de forme circulaire, et ce récipient 2 présente une ouverture supérieure (sur le haut) propre à être refermée par le couvercle 3.

Le moyen de filtration 4 est formé d'un caisson filtrant 41 qui se présente sous forme d'une cuvette ou de panier ouvert sur le dessus, du côté du couvercle 3. Autrement dit, ce caisson filtrant 41 présente une ouverture supérieure sur le dessus, qui est bordée par un anneau 45, et par exemple un anneau de forme circulaire. Ce caisson filtrant 41 comprend une paroi de fond 40 (situé du côté de la paroi de fond 20 du récipient 2) et un tamis périphérique 42 de forme tubulaire (et par exemple de forme cylindrique ou circulaire) tenu par des éléments de rigidification 43 reliant la paroi de fond 40 du caisson 41 à l'anneau 45. Le caisson filtrant 41 est mobile en rotation à l'intérieur du récipient 2.

Le couvercle 3 comprend une ouverture d'entrée 38 depuis laquelle est inséré le fluide sanguin. Le récipient 2 et le couvercle 3 délimitent entre eux une chambre d'aspiration du fluide sanguin. Une ouverture de sortie 22 formée sur la paroi latérale 21 du récipient 2 est prévue pour être reliée par une tubulure 7, dite première tubulure 7, à un dispositif d'aspiration du fluide sanguin 90 par l'intermédiaire d'une valve 9 sélective à double entrée.

Dans ce premier dispositif de filtration 1, le moyen de centrifugation comprend un moyen d'entraînement 5 couplé au moyen de filtration 4 pour un entraînement en rotation du moyen de filtration 4, et plus spécifiquement du caisson filtrant 41. Un arbre d'accouplement 50 du moyen d'entraînement 5 est prévu pour traverser la paroi de fond 20 du récipient 2 et être couplé en rotation au moyen de filtration 4, et en particulier couplé en rotation à la paroi de fond 40 du caisson filtrant 41. Pour cela, un orifice de réception 20a formé dans la paroi de fond 20 du récipient 2 est prévu pour le passage de l'arbre d'accouplement 50 du moyen d'entraînement 5.

Un joint d'étanchéité (non représenté) est prévu au niveau de l'orifice de réception 20a pour assurer la fermeture hermétique de la chambre d'aspiration du fluide sanguin.

L'ouverture d'entrée 38 du couvercle 3 peut être formée par une membrane élastique permettant l'insertion étanche d'un conduit d'aspiration 10 du fluide sanguin.

En variante, le couvercle 3 et le conduit d'aspiration 10 du fluide sanguin peuvent être formés d'une seule pièce.

Dans tous les cas, le conduit d'aspiration 10 peut être configuré pour recevoir une canule 80 d'aspiration du fluide sanguin.

La paroi de fond 40 du caisson filtrant 41 comporte en saillie un connecteur 46 recevant l'arbre d'accouplement 50 pour permettre le couplage en rotation du moyen d'entraînement 5 avec le moyen de filtration 4.

Selon le premier mode de réalisation illustré, le moyen d'entraînement 5 en rotation comprend une hélice 51 comprise dans un support 6 pour un guidage en rotation de l'hélice 51, le support 6 étant prévu pour être solidarisé au récipient 2, et en particulier à la paroi de fond 20 du récipient 2. L'hélice 51 comprend une roue 52 depuis laquelle s'étendent radialement des pales 53 et sur laquelle est solidarisé son arbre d'accouplement 50.

Bien entendu, le support comprend un manche s'étendant selon un axe de rotation et recevant la roue 52 de l'hélice 51 pour le guidage en rotation de l'hélice 51. Cet axe de rotation peut être colinéaire d'un axe de symétrie du cylindre dans lequel est inscrit le tamis périphérique 42.

Le support 6 est monté sur le récipient 2 par l'extérieur. En pratique, l'hélice 51 est disposée sur son support de guidage 6 puis le support de guidage 6 est rapproché du récipient 2 de sorte que l'arbre d'accouplement 50 de l'hélice 51 traverse l'orifice de réception 20a de la paroi de fond 20 du récipient 2 pour être couplé au connecteur 46 du moyen de filtration 4.

Le support 6 peut être solidarisé, préférentiellement de façon étanche, au récipient 2 par tout moyen de fixation, préférentiellement par serrage ou par ultrasons.

Selon le premier mode de réalisation illustré, le support 6 délimite avec la paroi de fond 20 du récipient 2 une chambre d'entraînement de l'hélice 51, la chambre d'aspiration du fluide sanguin et la chambre d'entraînement de l'hélice 51 étant hermétique l'une par rapport à l'autre.

La chambre d'entraînement de l'hélice 51 définie un volume pour l'aspiration d'un fluide d'air entre un orifice d'entrée d'air 60 et un orifice de sortie d'air 61 formés latéralement sur le support de guidage 6 de l'hélice 51. Les orifices d'entrée d'air 60 et de sortie d'air 61 du support 6 de l'hélice 51 sont avantageusement en regard l'un de l'autre pour permettre l'entraînement en rotation des pales 53 de l'hélice 51 par dépression d'air dans la chambre d'entraînement de l'hélice 51.

L'orifice de sortie d'air 61 est avantageusement relié par une deuxième tubulure 8 au dispositif d'aspiration par l'intermédiaire de la même valve 9 sélective.

La première tubulure 7 et la deuxième tubulure 8 sont chacune reliée à une entrée de la valve 9, alors que la sortie de la valve 9 est relié au dispositif d'aspiration.

La valve 9 est avantageusement configurée pour être commutée entre une première position dans laquelle la première tubulure 7 est reliée de façon fluidique à la sortie de la valve 9 et une deuxième position dans laquelle la deuxième tubulure 8 est reliée de façon fluidique à la sortie de la valve 9.

Le dispositif d'aspiration de fluide sanguin 90 est avantageusement une unité d'aspiration utilisée dans un bloc opératoire, comme par exemple une pompe fluidique. L'utilisation d'une unité d'aspiration d'un bloc opératoire permet l'utilisation aisée du premier dispositif de filtration 1 sans nécessité d'équipement supplémentaire dans le bloc opératoire pour son fonctionnement.

A titre d'exemple, ce dispositif d'aspiration de fluide sanguin 90 peut permet un débit massique de compris entre 4 et 8 bars.

On va décrire l'utilisation selon l'invention du premier dispositif de filtration 1.

Lors d'une opération de chirurgie au cours de laquelle il est pratiqué un fraisage d'un os. Le dispositif d'aspiration de fluide sanguin 90 est allumé et la valve 9 sélective commutée dans sa première position. Dans cette première position de la valve 9, le dispositif d'aspiration de fluide sanguin 90 est relié de façon fluidique à la chambre d'aspiration du fluide sanguin.

Pour rendre visible le champ opératoire du chirurgien, le fluide sanguin comprenant les résidus osseux est aspiré par la dépression d'air générée depuis l'ouverture d'entrée 38. Ce fluide sanguin est aspiré à l'aide de la canule 80 pour être introduit dans le récipient 2 via l'ouverture d'entrée 38. Le fluide sanguin s'écoule alors dans le moyen de filtration 4, autrement dit à l'intérieur du caisson filtrant 41, par l'anneau 45 du caisson filtrant 41.

La valve 9 sélective est alors commutée dans sa deuxième position. Dans cette deuxième position de la valve 9, le dispositif d'aspiration de fluide sanguin 90 est relié de façon fluidique à la chambre d'entraînement de l'hélice 51, la dépression d'air générée par la différence de pression entre les orifices d'entrée 60 et de sortie 61 du support 6 de l'hélice 51 permet l'entraînement en rotation des pales 53 de l'hélice 51.

La rotation de l'hélice 51 entraîne la rotation du caisson filtrant 41 du moyen de filtration 4. Par effet centrifuge, le flux sanguin comportant les résidus osseux est filtré de sorte que les résidus osseux soient retenus par les mailles du tamis périphérique 42 du moyen de filtration 4.

Ce mode de réalisation permet l'entraînement des pales 53 de l'hélice 51 à une vitesse de rotation comprise entre 50 000 tr/min et 80 000 tr/min.

La vanne 9 est commutée entre sa première position et sa deuxième position jusqu'à la filtration complète du fluide sanguin contenu dans le récipient 2.

Le dispositif d'aspiration de fluide sanguin 90 est alors éteint. Le couvercle 3 est enlevé du récipient 2 et le moyen de filtration 4 est retiré du récipient 2 en vue de collecter les résidus osseux.

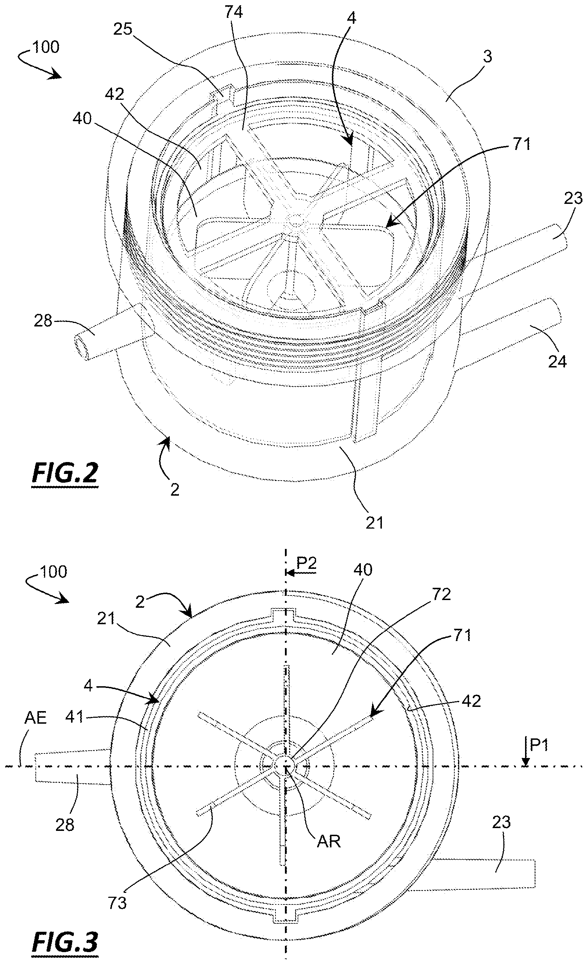

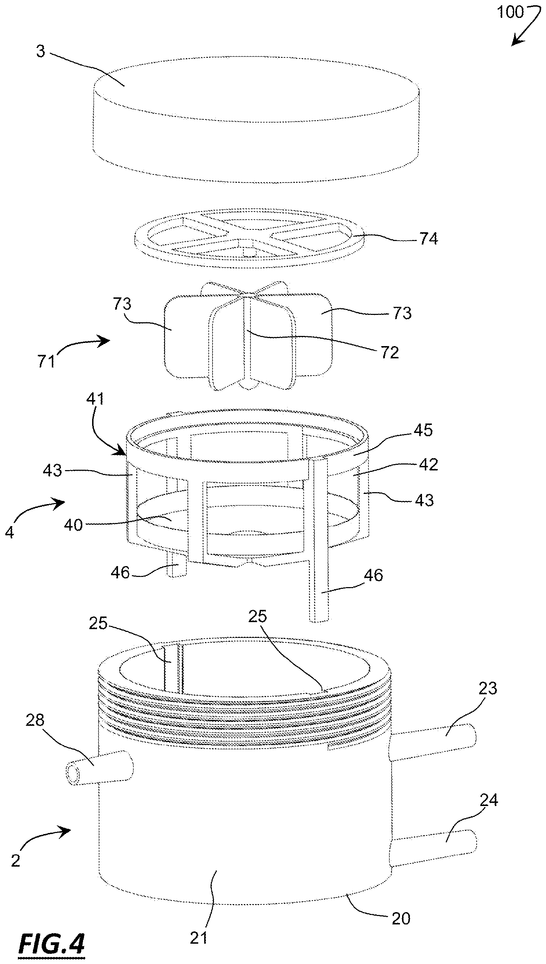

La suite de la description porte sur le second mode de réalisation des Figures 2 à 4, sur lesquelles est représenté un dispositif de filtration 100 (appelé par la suite second dispositif de filtration 100) pour une filtration d'un fluide sanguin comportant des résidus osseux selon un second mode de réalisation de l'invention.

Le second dispositif de filtration 100 comprend :
- un récipient 2 pour réceptionner le fluide sanguin,
- un couvercle 3 agencé pour une fermeture du récipient 2,
- un moyen de filtration 4 logé dans le récipient 2 pour la collecte des résidus osseux et
- un moyen de centrifugation conformé pour générer un effet centrifuge du fluide sanguin à l'intérieur du récipient 2.

Le récipient 2 est formé d'une paroi de fond 20 et d'une paroi latérale 21 (ou paroi périphérique) de forme circulaire, et ce récipient 2 présente une ouverture supérieure (sur le haut) propre à être refermée par le couvercle 3.

Le moyen de filtration 4 est formé d'un caisson filtrant 41 qui se présente sous forme d'une cuvette ou de panier ouvert sur le dessus, du côté du couvercle 3. Autrement dit, ce caisson filtrant 41 présente une ouverture supérieure sur le dessus, qui est bordée par un anneau 45, et par exemple un anneau de forme circulaire. Ce caisson filtrant 41 comprend une paroi de fond 40 (situé du côté de la paroi de fond 20 du récipient 2) et un tamis périphérique 42 de forme tubulaire (et par exemple de forme cylindrique ou circulaire) tenu par des éléments de rigidification 43 reliant la paroi de fond 40 du caisson 41 à l'anneau 45. la paroi de fond 40 du caisson filtrant 41 présente une forme générale de cuvette en creux, en particulier une cuvette de forme générale tronconique ou hémisphérique, et est donc oblique par rapport à l'axe de rotation AR et par rapport à l'horizontale. Cette paroi de fond 40 du caisson filtrant 41 présente plusieurs zones de tamisage, telles que des zones percées ou grillagées. Sur les Figures 2 à 4, les zones de tamisage de la paroi de fond 40 et celles également du tamis périphériques 42 ne sont pas illustrées.

Le caisson filtrant 41 est statique à l'intérieur du récipient 2, et il ne peut pas tourner dans le récipient 2. Pour ce faire, une ou plusieurs rainures 25 sont ménagées intérieurement dans la paroi latérale 21 du récipient 2, et le caisson filtrant 41 comprend un ou plusieurs rails 46 qui glissent dans la ou les rainures 25, interdisant ainsi le caisson filtrant 41 à tourner à l'intérieur du récipient 2. Le ou les rails 45 permettent aussi que la paroi de fond 40 du caisson filtrant 41 soit surélevée ou écartée vis-à-vis de la paroi de fond 20

Le récipient 2 comprend une ouverture d'entrée 28 depuis laquelle est inséré le fluide sanguin. Cette ouverture d'entrée 28 est ménagée dans la paroi latérale 21 du récipient 2 en regard d'une fenêtre (non visible) ménagée dans le tamis périphérique 42. Ainsi, le fluide sanguin comportant des résidus osseux entre par cette ouverture d'entrée 28 et entre à l'intérieur du caisson filtrant 41 en passant à travers cette fenêtre ménagée dans le tamis périphérique 42.

L'ouverture d'entrée 28 peut être formée par une membrane élastique permettant l'insertion étanche d'un conduit d'aspiration (non illustré sur les Figures 2 à 4) du fluide sanguin. En variante, le récipient 2 et le conduit d'aspiration du fluide sanguin peuvent être formés d'une seule pièce. Dans tous les cas, le conduit d'aspiration peut être configuré pour recevoir une canule (non illustrée sur les Figures 2 à 4) d'aspiration du fluide sanguin.

Le récipient 2 et le couvercle 3 délimitent entre eux une chambre d'aspiration du fluide sanguin. Une ouverture de sortie supérieure 23 formée sur la paroi latérale 21 du récipient 2, au-dessus de la paroi de fond 40 du caisson filtrant 41, est prévue pour être reliée par une tubulure, dite première tubulure, à un dispositif d'aspiration du fluide sanguin par l'intermédiaire d'une valve à double entrée.

Une ouverture de sortie inférieure 24 formée sur la paroi latérale 21 du récipient 2, en-dessous de la paroi de fond 40 du caisson filtrant 41, est prévue pour être reliée par une tubulure, dite seconde tubulure, au dispositif d'aspiration du fluide sanguin par l'intermédiaire de la même valve à double entrée. La première tubulure et la deuxième tubulure sont chacune reliée à une entrée de la valve, alors que la sortie de la valve est reliée au dispositif d'aspiration de fluide sanguin. La valve est avantageusement configurée pour reliée de façon fluidique les deux ouvertures de sortie 23, 24 en même temps au dispositif d'aspiration de fluide sanguin.

Dans ce second dispositif de filtration 100, le moyen de centrifugation comprend une hélice 71 et comprend également le dispositif d'aspiration du fluide sanguin qui entraine en rotation cette hélice 71, où l'hélice 71 est disposée à l'intérieur du caisson filtrant 41 en étant entourée par le tamis périphérique 42 et l'hélice 71 est montée rotative selon un axe de rotation AR. Cet axe de rotation AR peut être colinéaire d'un axe de symétrie du cylindre dans lequel est inscrit le tamis périphérique 42.

L'hélice 71 comprend un arbre central 72 centré sur l'axe de rotation AR et depuis lequel s'étendent radialement des pales 73, où cet arbre central 72 présente :
- une extrémité inférieure montée pivotante sur la paroi de fond 40 du caisson filtrant 41;
- une extrémité supérieure montée pivotante sur un plateau supérieur 74 fixé sur le tamis périphérique 42 du caisson filtrant 41, et plus précisément fixé sur l'anneau 45.

L'ouverture d'entrée 28 s'étend selon un axe d'entrée AE intersectant l'axe de rotation AR de l'hélice 71 et formant avec cet axe de rotation AR un premier plan médian P1, ce premier plan médian P1 étant orthogonal à un second plan médian P2 incluant l'axe de rotation AR. Les ouvertures de sortie 23, 24 sont déportées par rapport au premier plan médian P1, de sorte qu'elles ne soient pas dans l'alignement de l'axe d'entrée AE et soient donc excentrées. Par ailleurs, l'ouverture d'entrée 28 et les ouvertures de sortie 23, 24 sont disposées de part et d'autre du second plan médian P2, pour permettre l'entraînement en rotation des pales 53 de l'hélice 51 par dépression d'air dans la chambre d'entraînement de l'hélice 51.

On va décrire l'utilisation selon l'invention du second dispositif de filtration 100.

Le dispositif d'aspiration de fluide sanguin 90 est allumé et la valve 9 assure que le dispositif d'aspiration de fluide sanguin 90 soit relié de façon fluidique à la chambre d'aspiration du fluide sanguin, via les deux ouvertures de sortie 23, 24. Ainsi, le fluide sanguin comprenant les résidus osseux est aspiré par la dépression d'air générée depuis l'ouverture d'entrée 28. Ce fluide sanguin est aspiré à l'aide de la canule (non illustrée) pour être introduit dans le récipient 2 via l'ouverture d'entrée 28. Le fluide sanguin s'écoule alors dans le moyen de filtration 4, autrement dit à l'intérieur du caisson filtrant 41, par la fenêtre ménagée dans le tamis périphérique 42.

La dépression d'air générée par la différence de pression entre l'ouverture de sortie supérieure 23 et l'ouverture d'entrée 28 permet l'entraînement en rotation des pales 73 de l'hélice 71. La rotation de l'hélice 71 procure un effet centrifuge à l'intérieur du caisson filtrant 41, et le flux sanguin comportant les résidus osseux est filtré de sorte que les résidus osseux soient retenus par les mailles du tamis périphérique 42 du caisson filtrant 41, et sédimentent dans la paroi de fond 40 en forme de cuvette. Le fluide sanguin filtré s'écoule quant à lui préférentiellement par l'ouverture de sortie inférieure 24.

Le dispositif d'aspiration de fluide sanguin est activé jusqu'à la filtration complète du fluide sanguin contenu dans le récipient 2, et ensuite le dispositif d'aspiration de fluide sanguin est éteint. Le couvercle 3 est enlevé du récipient 2 et le moyen de filtration 4 est retiré du récipient 2 en vue de collecter les résidus osseux.

Bien entendu, la présente invention n'est pas limitée aux modes de réalisation qui viennent d'être décrits. En particulier, diverses modifications et variantes peuvent être envisagées par l'homme du métier sans sortir du cadre de l'invention tel que défini dans la revendication 1. A titre d'exemple, on pourra prévoir que le moyen d'entraînement du premier dispositif de filtration est une manivelle ou un moteur couplé à l'arbre d'accouplement pour permettre l'entraînement en rotation du moyen de filtration.

## Revendications

1. Dispositif de filtration (1; 100) pour une filtration d'un fluide sanguin comportant des résidus osseux, ledit dispositif de filtration (1) comprenant :
- un récipient (2) et un couvercle (3) agencé pour une fermeture du récipient (2), ledit récipient (2) et ledit couvercle (3) délimitant entre eux une chambre d'aspiration du fluide sanguin dans laquelle débouchent au moins une ouverture de sortie (22 ; 23, 24) apte à être reliée à un dispositif d'aspiration du fluide sanguin (90), et une ouverture d'entrée (38 ; 28) pour une entrée du fluide sanguin comportant des résidus osseux ;
- un moyen de filtration (4) logé dans le récipient (2) pour la collecte des résidus osseux,
ledit dispositif de filtration (1 ; 100) étant **caractérisé en ce que** le moyen de filtration (4) est pourvu d'un caisson filtrant (41) qui comprend une paroi de fond (40) et un tamis périphérique (42) à travers lequel le fluide sanguin est filtré et par lequel les résidus osseux sont retenus ;
et **en ce qu'**il comprend un moyen de centrifugation conformé pour générer un effet centrifuge du fluide sanguin à l'intérieur du caisson filtrant (41).

2. Dispositif de filtration (1 ; 100) selon la revendication 1, dans lequel le moyen de centrifugation comprend une hélice (51; 71) et le dispositif d'aspiration du fluide sanguin (90) qui entraine en rotation ladite hélice (51 ; 71).

3. Dispositif de filtration (1) selon l'une quelconque des revendications 1 et 2, dans lequel le moyen de centrifugation comprend un moyen d'entraînement (5) couplé au moyen de filtration (4) pour un entraînement en rotation du moyen de filtration (4).

4. Dispositif de filtration (1) selon les revendications 2 et 3, dans lequel l'hélice (51) est couplée en rotation au caisson filtrant (41) de sorte que le moyen d'entraînement (5) entraîne en rotation le caisson filtrant (41).

5. Dispositif de filtration (1) selon la revendication 4, dans lequel le moyen d'entraînement (5) comprend un arbre d'accouplement (50) traversant une paroi de fond (20) du récipient (2) pour être couplé en rotation, d'une part, au caisson filtrant (41) et, d'autre part, à l'hélice (51).

6. Dispositif de filtration (1) selon la revendication 5, dans lequel l'hélice (51) est comprise dans un support (6) pour un guidage en rotation de l'hélice (51), le support (6) étant solidarisé au récipient (2).

7. Dispositif de filtration (1) selonla revendication 6, dans lequel le support (6) délimite une chambre d'entraînement de l'hélice (51), la chambre d'aspiration du fluide sanguin et la chambre d'entraînement étant hermétique l'une par rapport à l'autre.

8. Dispositif de filtration (1) selon la revendication 7, dans lequel le support (6) comporte latéralement un orifice d'entrée d'air (60) et un orifice de sortie d'air (61) en regard l'un de l'autre pour permettre l'entraînement en rotation de l'hélice (51), l'orifice de sortie d'air (61) étant relié au dispositif d'aspiration du fluide sanguin (90).

9. Dispositif de filtration (1) selon l'une quelconque des revendications 3 à 8, dans lequel l'ouverture d'entrée (38) est ménagée sur le couvercle (3).

10. Dispositif de filtration (100) selon la revendication 2, dans lequel l'hélice (71) est disposée à l'intérieur du caisson filtrant (41) en étant entourée par le tamis périphérique (42) et l'hélice (71) est montée rotative selon un axe de rotation (AR).

11. Dispositif de filtration (100) selon la revendication 10, dans lequel le caisson filtrant (41) est statique à l'intérieur du récipient (2) et l'ouverture d'entrée (28) est ménagée sur le récipient (2) en regard d'une fenêtre ménagée dans le tamis périphérique (42).

12. Dispositif de filtration (100) selon la revendication 11, dans lequel l'ouverture d'entrée (28) s'étend selon un axe d'entrée (AE) intersectant l'axe de rotation (AR) de l'hélice (71) et formant avec ledit axe de rotation (AR) un premier plan médian (P1), ledit premier plan médian (P1) étant orthogonal à un second plan médian (P2) incluant l'axe de rotation (AR), et la ou les ouvertures de sortie (23 ; 24) sont ménagées sur le récipient (2) en étant déportées par rapport au premier plan médian (P1), avec l'ouverture d'entrée (28) et la ou les ouvertures de sortie (23 ; 24) disposées de part et d'autre du second plan médian (P2).

13. Dispositif de filtration (1) selon l'une quelconque des revendications 10 à 12, dans lequel sont prévues deux ouvertures de sortie (23, 24), dont une ouverture de sortie supérieure (23) disposée au-dessus de la paroi de fond (40) du caisson filtrant (41), et une ouverture de sortie inférieure (24) disposée en-dessous de la paroi de fond (40) du caisson filtrant (41).

14. Dispositif de filtration (1) selon l'une quelconque des revendications 10 à 13, dans lequel l'hélice (71) comprend un arbre central (72) centré sur l'axe de rotation et depuis lequel s'étendent radialement des pales (73), où ledit arbre central (72) présente une extrémité inférieure montée pivotante sur la paroi de fond (40) du caisson filtrant (41).

15. Dispositif de filtration (1) selon la revendication 14, dans lequel l'arbre central (72) de l'hélice (71) présente une extrémité supérieure montée pivotante sur un plateau supérieur (74) fixé sur le tamis périphérique (42) du caisson filtrant (41).

## Patentansprüche

1. Filtervorrichtung (1; 100) für ein Filtern eines Knochenrückstände umfassenden Blutfluids, wobei die Filtervorrichtung (1) umfasst:
- einen Behälter (2) und einen Deckel (3), der für ein Verschließen des Behälters (2) eingerichtet ist, wobei der Behälter (2) und der Deckel (3) zwischen sich eine Kammer zum Ansaugen des Blutfluids begrenzen, in der mindestens eine Austrittsöffnung (22; 23, 24), die mit einer Vorrichtung zum Ansaugen des Blutfluids (90) verbunden werden kann, und eine Eintrittsöffnung (38; 28) für einen Eintritt des Knochenrückstände umfassenden Blutfluids münden;
- ein in dem Behälter (2) untergebrachtes Filtermittel (4) zum Sammeln der Knochenrückstände,
wobei die Filtervorrichtung (1; 100) **dadurch gekennzeichnet ist, dass** das Filtermittel (4) mit einem Filtergehäuse (41) versehen ist, das eine Bodenwand (40) und ein umlaufendes Sieb (42) umfasst, durch das das Blutfluid gefiltert wird und von dem die Knochenrückstände zurückgehalten werden;
und dadurch, dass sie ein Zentrifugiermittel umfasst, das so ausgestaltet ist, dass es einen Zentrifugaleffekt für das Blutfluid im Inneren des Filtergehäuses (41) erzeugt.

2. Filtervorrichtung (1; 100) nach Anspruch 1, wobei das Zentrifugiermittel eine Schraube (51; 71) und die Vorrichtung zum Ansaugen des Blutfluids (90), die die Schraube (51; 71) drehend antreibt, umfasst.

3. Filtervorrichtung (1) nach einem der Ansprüche 1 und 2, wobei das Zentrifugiermittel ein mit dem Filtermittel (4) gekoppeltes Antriebsmittel (5) für einen Drehantrieb des Filtermittels (4) umfasst.

4. Filtervorrichtung (1) nach den Ansprüchen 2 und 3, wobei die Schraube (51) so mit dem Filtergehäuse (41) drehgekoppelt ist, dass das Antriebsmittel (5) das Filtergehäuse (41) drehend antreibt.

5. Filtervorrichtung (1) nach Anspruch 4, wobei das Antriebsmittel (5) eine Kopplungswelle (50) umfasst, die durch eine Bodenwand (20) des Behälters (2) hindurchgeht, um einerseits mit dem Filtergehäuse (41) und andererseits mit der Schraube (51) drehgekoppelt zu werden.

6. Filtervorrichtung (1) nach Anspruch 5, wobei die Schraube (51) in einer Halterung (6) für eine Drehführung der Schraube (51) umfasst ist, wobei die Halterung (6) fest mit dem Behälter (2) verbunden ist.

7. Filtervorrichtung (1) nach Anspruch 6, wobei die Halterung (6) eine Antriebskammer der Schraube (51) begrenzt, wobei die Kammer zum Ansaugen des Blutfluids und die Antriebskammer in Bezug zueinander hermetisch abgedichtet sind.

8. Filtervorrichtung (1) nach Anspruch 7, wobei die Halterung (6) seitlich eine Lufteintrittsöffnung (60) und eine Luftaustrittsöffnung (61) umfasst, die einander gegenüberliegen, um den Drehantrieb der Schraube (51) zu ermöglichen, wobei die Luftaustrittsöffnung (61) mit der Vorrichtung zum Ansaugen des Blutfluids (90) verbunden ist.

9. Filtervorrichtung (1) nach einem der Ansprüche 3 bis 8, wobei die Eintrittsöffnung (38) am Deckel (3) ausgebildet ist.

10. Filtervorrichtung (100) nach Anspruch 2, wobei die Schraube (71) im Inneren des Filtergehäuses (41) angeordnet ist, wobei sie von dem umlaufenden Sieb (42) umgeben ist, und die Schraube (71) um eine Drehachse (AR) drehbar angebracht ist.

11. Filtervorrichtung (100) nach Anspruch 10, wobei das Filtergehäuse (41) im Inneren des Behälters (2) statisch ist, und die Eintrittsöffnung (28) einem im umlaufenden Sieb (42) ausgebildeten Fenster gegenüberliegend am Behälter (2) ausgebildet ist.

12. Filtervorrichtung (100) nach Anspruch 11, wobei sich die Eintrittsöffnung (28) entlang einer Eintrittsachse (AE) erstreckt, die die Drehachse (AR) der Schraube (71) schneidet und mit der Drehachse (AR) eine erste Mittelebene (P1) bildet, wobei die erste Mittelebene (P1) zu einer zweiten Mittelebene (P2), die die Drehachse (AR) enthält, orthogonal ist, und die Austrittsöffnung oder die Austrittsöffnungen (23; 24) in Bezug auf die erste Mittelebene (P1) versetzt am Behälter (2) ausgebildet sind, wobei die Eintrittsöffnung (28) und die Austrittsöffnung oder die Austrittsöffnungen (23; 24) beiderseits der zweiten Mittelebene (P2) angeordnet sind.

13. Filtervorrichtung (1) nach einem der Ansprüche 10 bis 12, wobei zwei Austrittsöffnungen (23, 24) vorgesehen sind, darunter eine obere Austrittsöffnung (23), die über der Bodenwand (40) des Filtergehäuses (41) angeordnet ist, und eine untere Austrittsöffnung (24), die unter der Bodenwand (40) des Filtergehäuses (41) angeordnet ist.

14. Filtervorrichtung (1) nach einem der Ansprüche 10 bis 13, wobei die Schraube (71) eine Zentralwelle (72) umfasst, die auf der Drehachse zentriert ist und von der sich radial Schaufeln (73) erstrecken, wobei die Zentralwelle (72) ein unteres Ende aufweist, das schwenkbar an der Bodenwand (40) des Filtergehäuses (41) angebracht ist.

15. Filtervorrichtung (1) nach Anspruch 14, wobei die Zentralwelle (72) der Schraube (71) ein oberes Ende aufweist, das schwenkbar an einer am umlaufenden Sieb (42) des Filtergehäuses (41) befestigten oberen Platte (74) angebracht ist.

## Claims

1. A filtration device (1; 100) for a filtration of a blood fluid including bone residues, said filtration device (1) comprising:
- a container (2) and a lid (3) arranged to close the container (2), said container (2) and said lid (3) delimiting between them a blood fluid suction chamber into which at least one outlet opening (22; 23, 24) adapted to be connected to a blood fluid aspiration device (90), and an inlet opening (38; 28) for an inlet of blood fluid including bone residues open;
- a filtration means (4) housed in the container (2) for the collection of bone residues,
said filtration device (1; 100) being **characterized in that** the filtration means (4) is provided with a filter box (41) which comprises a bottom wall (40) and a peripheral screen (42) through which the blood fluid is filtered and by which the bone residues are retained;
and **in that** it comprises centrifugation means configured to generate a centrifugal effect of the blood fluid inside the filter box (41).

2. The filtration device (1; 100) according to claim 1, wherein the centrifugation means comprises a propeller (51; 71) and the blood fluid aspiration device (90) which rotatably drives said propeller (51; 71).

3. The filtration device (1) according to any one of claims 1 and 2, wherein the centrifugation means comprises drive means (5) coupled to the filtration means (4) for a rotatably driving of the filtration means (4).

4. The filtration device (1) according to claims 2 and 3, wherein the propeller (51) is coupled in rotation to the filter box (41) so that the drive means (5) rotatably drives the filter box (41).

5. The filtration device (1) according to claim 4, wherein the drive means (5) comprises a coupling shaft (50) passing through a bottom wall (20) of the container (2) to be coupled in rotation, on the one hand, to the filter box (41) and, on the other hand, to the propeller (51).

6. The filtration device (1) according to claim 5, wherein the propeller (51) is comprised in a support (6) for a rotatably guiding of the propeller (51), the support (6) being secured to the container (2).

7. The filtration device (1) according to claim 6, wherein the support (6) defines a propeller driving chamber (51), the blood fluid suction chamber and the driving chamber being sealed relative to one another.

8. The filtration device (1) according to claim 7, wherein the support (6) laterally includes an air inlet orifice (60) and an air outlet orifice (61) facing each other to allow the rotatably driving of the propeller (51), the air outlet orifice (61) being connected to the blood fluid suction device (90).

9. The filtration device (1) according to any one of claims 3 to 8, wherein the inlet opening (38) is provided on the lid (3).

10. The filtration device (100) according to claim 2, wherein the propeller (71) is arranged inside the filter box (41) by being surrounded by the peripheral screen (42) and the propeller (71) is rotatably mounted around an axis of rotation (AR).

11. The filtration device (100) according to claim 10, wherein the filter box (41) is static inside the container (2) and the inlet opening (28) is provided on the container (2) opposite a window formed in the peripheral screen (42).

12. The filtration device (100) according to claim 11, wherein the inlet opening (28) extends along an inlet axis (AE) intersecting the axis of rotation (AR) of the propeller (71) and forming with said axis of rotation (AR) a first median plane (P1), said first median plane (P1) being orthogonal to a second median plane (P2) including the axis of rotation (AR), and the outlet opening(s) (23; 24) are provided on the container (2) by being offset relative to the first median plane (P1), with the inlet opening (28) and the outlet opening(s) (23; 24) arranged on either side of the second median plane (P2).

13. The filtration device (1) according to any one of claims 10 to 12, wherein two outlet openings (23, 24) are provided, including an upper outlet opening (23) arranged above the wall of the bottom (40) of the filter box (41), and a lower outlet opening (24) arranged below the bottom wall (40) of the filter box (41).

14. The filtration device (1) according to any one of claims 10 to 13, wherein the propeller (71) comprises a central shaft (72) centered on the axis of rotation and from which blades (73) radially extend, where said central shaft (72) has a lower end pivotally mounted on the bottom wall (40) of the filter box (41).

15. The filtration device (1) according to claim 14, wherein the central shaft (72) of the propeller (71) has an upper end pivotally mounted on an upper plate (74) fixed to the peripheral screen (42) of the filter box (41).
